(19) Europäisches Patentamt
European Patent Office
Office européen des brevets

(11) EP 0 997 452 B1

(12) FASCICULE DE BREVET EUROPEEN

(45) Date de publication et mention de la délivrance du brevet:
**29.01.2003 Bulletin 2003/05**

(51) Int Cl.[7]: **C07C 49/553**, C07C 33/02, C07C 45/69, C07C 45/66, C11B 9/00

(21) Numéro de dépôt: **99120446.2**

(22) Date de dépôt: **14.10.1999**

(54) **Nouvelles cétones bicycliques et leur utilisation dans le domaine de la parfumerie**

Bicyclische Ketone und ihre Verwendung als Riechstoff

Bicyclic ketones and their use as perfume

(84) Etats contractants désignés:
**AT BE CH CY DE DK ES FI FR GB GR IE IT LI LU MC NL PT SE**

(30) Priorité: **26.10.1998 CH 982155**

(43) Date de publication de la demande:
**03.05.2000 Bulletin 2000/18**

(73) Titulaire: **FIRMENICH SA**
**1211 Genève 8 (CH)**

(72) Inventeur: **Giersch, Wolfgang Klaus**
**1233 Bernex (CH)**

(74) Mandataire:
**Salvaterra-Garcia, Maria de Lurdes, Dr.**
**Firmenich SA**
**Case Postale 239**
**1211 Genève 8 (CH)**

(56) Documents cités:
**EP-A- 0 116 277**
**DE-B- 1 045 393**
**US-A- 3 076 022**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a trait à des composés nouveaux, utiles dans le domaine de la parfumerie. Elle concerne plus particulièrement les composés de formule générale

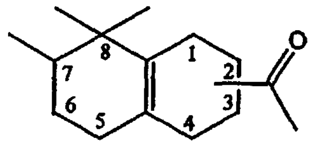

(I)

dans laquelle le groupe substituant acétyle se trouve en position 2 ou 3 du cycle, ou tout mélange de composés de formule (I). Toute référence ultérieure au composé de formule (I) désignera donc aussi bien l'un ou l'autre des isomères, ou un mélange de ceux-ci.

Les composés de la présente invention sont nouveaux. Nous avons découvert qu'ils possèdent des qualités odorantes très utiles et appréciées et qu'ils peuvent, de ce fait, servir à préparer des parfums, compositions parfumantes et articles parfumés. Ils sont employés pour conférer notamment des effets odorants de type cuir naturel.

L'utilisation en parfumerie de composés de structures proches de celui de la présente invention fait partie de l'état de la technique. On peut citer les brevets US 3,929,677 et EP 743 297 qui divulguent de tels composés et font état de leurs propriétés odorantes.

D'une façon très surprenante, nous avons découvert que les composés de formule (I) présentent dans leur parfum un caractère tout à fait original par rapport à celui des composés connus. Il s'agit en effet d'un ensemble de notes boisées, poudrées-violette ambre gris, un peu algue marine, accompagné d'un côté cuir très naturel, cuir frais de type daim ("suède") tout à fait inattendu. Cette dernière connotation a pour effet de distinguer très nettement le composé de la présente invention des produits de structure similaire connus de l'état de la technique et lui confère une grande valeur pour son utilisation en parfumerie.

Les composés se prêtent aussi bien à une utilisation en parfumerie fine, dans les parfums, eaux de toilette ou lotions après-rasage, qu'à d'autres emplois courants en parfumerie tels que le parfumage de savons, gels de douche ou de bain, de produits d'hygiène ou de traitement des cheveux comme les shampoings, ainsi que de déodorants corporels et déodorants ambiants, ou encore de préparations cosmétiques.

Les composés (I) peuvent également être employés dans des applications telles que détergents liquides ou solides destinés au traitement des textiles, adoucissants textiles, ou encore compositions détergentes ou produits d'entretien destinés au nettoyage de la vaisselle ou de surfaces variées.

Dans ces applications, les composés de l'invention peuvent être utilisés seuls ou en mélange avec d'autres ingrédients parfumants, des solvants ou adjuvants d'usage courant en parfumerie. La nature et la variété de ces coingrédients n'appellent pas une description plus détaillée ici, qui ne saurait d'ailleurs être exhaustive, l'homme du métier étant à même de les choisir de par ses connaissances générales et en fonction de la nature du produit à parfumer et de l'effet olfactif recherché.

Ces ingrédients parfumants appartiennent à des classes chimiques aussi variées que les alcools, aldéhydes, cétones, esters, ethers, acétates, nitriles, hydrocarbures terpéniques, composés hétérocycliques azotés ou soufrés, ainsi que des huiles essentielles d'origine naturelle ou synthétiques. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles les composés selon l'invention peuvent être incorporés dans les produits divers sus-mentionnés varient dans une gamme de valeurs étendue. Ces valeurs dépendent de la nature de l'article ou produit que l'on veut parfumer et de l'effet olfactif recherché, ainsi que de la nature des coingrédients dans une composition donnée lorsque les composés de l'invention sont utilisés en mélange avec des coingrédients parfumants, des solvants ou des adjuvants d'usage courant dans l'art.

A titre d'exemple, on peut citer des concentrations typiques de l'ordre de 5 à 10% en poids, voire même 20% en poids, des composés de l'invention, par rapport au poids de composition parfumante dans laquelle ils sont incorporés. Des concentrations bien inférieures à celles-ci peuvent être utilisées lorsque les composés sont directement appliqués dans le parfumage des produits de consommation divers cités auparavant.

L'invention concerne également un procédé de préparation des composés de formule (I). Selon le procédé de l'invention, on soumet un alcool de formule suivante

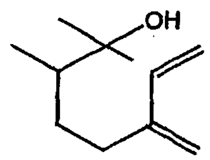

(II)

à une réaction de Diels Alder avec une méthyle vinyle cétone, suivie d'une déshydratation et d'une cyclisation, trois étapes réalisées en cascade, dans un seul pot («one-pot reaction»).

L'alcool de formule (II) est un composé nouveau qui fait également l'objet de l'invention. Ce dernier peut être obtenu par une réaction dite de Grignard, à partir d'époxymyrcène.

L'ensemble de la synthèse peut ainsi être schématisé de la façon suivante

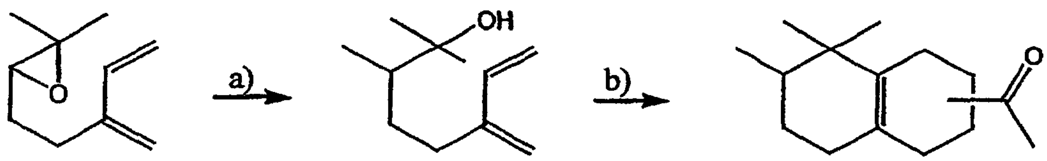


a) $CH_3MgCl$/tétrahydrofurane, catalyseur CuBr, (iso-$C_3H_9)_2O$
b) But-1-én-3-one, éthyl-éther de $BF_3$, toluène

Les conditions de réaction sont décrites en détail dans un exemple présenté plus loin.

L'invention sera maintenant décrite de façon plus détaillée dans les exemples suivants, dans lesquels les températures sont indiquées en degrés centigrades et les abréviations ont le sens usuel dans l'art.

Exemple 1

Préparation de la 1-(1,2,3,4,5,6,7,8-octahydro-7,8,8-triméthyl-2-naphtalényl)-1-éthanone

a) On a ajouté 1230 ml d'une solution 3M de $CH_3MgCl$ dans du tétrahydrofurane (Fluka), à 26,5 g de CuBr (Fluka) dans 1 1 d'éther diisopropylique, avec agitateur, à O° (température du bain). Après une nuit, on a refroidit le mélange réactionnel à -20° et on a ajouté goutte à goutte 467 g d'époxymyrcène [2-(3,4-époxy-4-méthylpentyl)-1,3-butadiène] dans 41 d'éther isopropylique. Après l'addition, on a réchauffé le mélange réactionnel jusqu'à température ambiante. Après 23 h, la réaction était achevée. L'hydrolyse à l'aide d'une solution aqueuse de $NH_4Cl$ à 15% et un lavage à la saumure a fourni un produit brut que l'on a distillé pour obtenir le 2,3-diméthyl-6-méthylène-7-octén-2-ol à 96,6% pur. Le rendement était de 453,7 g (88%).
Point d'ébullition : 75°/40 Pa
Données spectrales du 2,3-diméthyl-6-méthylène-7-octén-2-ol
RMN($^1$H): 0,95(d, J=6,8, 3H) ; 1,16 et 1,17(2s, 6H) ; 5,01(s, 2H) ; 5,06(d, J=11,2, 1H) ; 5,23(d, J=17,6, 1H); 6,37 (dd, $J_1$=11,2, $J_2$=17,6, 1H)
RMN($^{13}$C): 14,5(q); 26,2(q); 27,1(q); 30,1(t); 30,3(t); 44,2(d); 73,4(s); 113,2(t) ; 115,7(t) ; 138,9(d) ; 146,6(s)
SM : 168($M^+$, 0), 153(1), 150(4), 135(9), 107(7), 95(12), 81(21), 68(49), 59(100), 41(37)

b) On a ajouté à une solution de produit obtenu sous a) (300 g), de la but-1-en-3-one (147,5 g ; Fluka) et du toluène (3 1), avec de l'éthyl-éther de $BF_3$ (30 ml), sous agitation. La réaction exothermique a fait passer le mélange réactionnel de 23° à 33°. On l'a ensuite chauffé à 40°. Après 22 h, la réaction de Diels-Alder était achevée. On a ajouté à nouveau du $BF_3$.éthyl-éther à 40°; le mélange réactionnel est devenu brun puis brun foncé après 45 h. Le mélange réactionnel a alors été lavé à l'aide d'une solution à 10% de NaOH et avec de la saumure, puis distillé pour fournir le produit désiré sous forme d'un mélange de composés contenant la 1-(1,2,3,4,5,6,7,8-octahydro-7,8,8-triméthyl-2-naphtalenyl)-1-éthanone (2 diastéréomères) et la 1-(1,2,3,4,5,6,7,8-octahydro-5,5,6-triéthyl-2-naphtalenyl)-1-éthanone. Le rendement était de 258,2 g (65%).

Données spectrales des produits qui ont pu être élucidés après séparation du mélange par chromatographie en phase gazeuse :

Cis-1-(1,2,3,4,5,6,7,8-octahydro-7,8,8-triméthyl-2-naphtalenyl)-1-éthanone RMN($^1$H) : 0,88(d, J=6, 3H) ; 0,89(s) 1,02(s) ; 2,17(s)
RMN($^{13}$C) : 16,4(q) ; 22,7(q) ; 25,2(t); 26,6(t) ; 26,9(t); 27,8(q) ; 28,4(q) ; 29,2(t) ; 30,5(t); 37,2(s); 39,3(d); 48,8(d); 126,8(s); 132,8(s); 212,6(s)
SM: 220($M^+$, 35), 205(40), 177(57), 161(14), 135(20), 119(23), 107(38), 91(69), 43(100)

Trans-1-(1,2,3,4,5,6,7,8-octahydro-7,8,8-triméthyl-2-naphtalenyl)-1-éthanone
RMN($^1$H): 0,89(d, J=6, 3H); 0,82 et 0,97(2s, 6H); 2,19(s, 3H)
RMN($^{13}$C): 17,0(q); 20,5(q); 25,5(t); 25,7(q); 27,2(t); 27,6(t); 28,5(q); 31,1(2t); 39,7(d); 49,2(d); 127,2(s); 134,2(s); 213,0 (s)
SM: 220($M^+$, 38), 205(42), 177(57), 161(14), 135(22), 119(24), 107(41), 91(67), 43(100)
Cis-1-(2-isopropyl-2-méthyl-1-oxaspiro[4.5]dec-8-yl)-1-éthanone
RMN($^1$H): 0,84 et 0,92(2d, J=6,8, 6H); 1.06(s, 3H); 2,13(s, 3H) RMN($^{13}$C): 17,9(q); 18,4(q); 23,3(q); 24,6(t); 25,0(t); 27,6(q); 34,4(t); 37,2(t); 37,9(t); 38,2(t), 38,4(d); 51,1(d); 80,2(s); 85,9(s); 212,2(s)
SM: 238($M^+$, 1), 223(3), 195(100), 177(41), 153(30), 135(30), 119(17), 93(12), 83(12), 43(23)
Trans-1-(2-isopropyl-2-méthyl-1-oxaspiro[4.5]dec-8-yl)-1-éthanone
RMN($^1$H) : 0,86 et 0,92(2d, J=6,8, 6H); 1,11(s, 3H) ; 2,14(s, 3H)
RMN($^{13}$C): 17,9(q); 18,4(q); 23,8(q); 26,5(2t); 28,3(q); 34,19(t); 34,31(t); 37,2(t) ; 37,9(d) ; 38,6(t) ; 50,2(d) ; 82,5(s) ; 85,4(s) ; 211,9(s)
SM: 238($M^+$, 0,5), 223(1), 195(60), 177(23), 153(21), 135(22), 119(14), 93(13), 83(16), 71(17), 43(100)

Exemple 2

Composition parfumante

On a préparé une composition parfumante de base pour une eau de toilette féminine de type fleurie-herbacée à partir des ingrédients suivants :

| Ingrédients | Parties en poids |
|---|---|
| Acétate d'hexyle | 20 |
| Acétate de benzyle | 150 |
| Acétate de carbinol | 20 |
| Acétate de citronellyle | 80 |
| Acétate de géranyle | 180 |
| Acétate de linalyle | 25 |
| Acétate de phényléthyle | 50 |
| Acétate de cis-3-hexénol à 1% * | 40 |
| Aldéhyde anisique à 10% * | 130 |
| Allyl amyl glycolate à 10% * | 20 |
| Ambrettolide ® 1) à 10% * | 80 |
| Anthranilate de méthyle | 10 |
| Cetalox ® 2) à 10% * | 25 |
| Cis-3-hexénol à 1% * | 40 |
| Citronellol | 80 |
| Dihydromyrcenol | 75 |
| β-Dorinone ® 3) à 10% * | 25 |
| Ethylvanilline à 10% * | 40 |
| Eugénol | 20 |

* dans le dipropylène glycol (DIPG)
1) 9-hexadécén-16-olide ; origine : Givaudan-Roure SA, Vernier, Suisse
2) 8,12-époxy-13,14,15,16-tétranorlabdane; origine : Firmenich SA, Genève, Suisse
3) β-damascone ; origine : Firmenich SA, Genève, Suisse

(suite)

| Ingrédients | Parties en poids |
|---|---|
| Exaltolide ® [4)] | 170 |
| Firsantol ® [5)] | 10 |
| Floralozone ® [6)] à 10% * | 20 |
| Galaxolide ® [7)] 50 | 730 |
| Géraniol | 10 |
| Hédione ® [8)] | 600 |
| Hélional ® [9)] | 10 |
| Indol à 10% * | 10 |
| Iralia ® total [10)] | 10 |
| Liffarome ® [11)] à 10% * | 10 |
| Lilial ® [12)] | 260 |
| Lyral ® [13)] | 30 |
| Oxyde de rose à 1% * | 50 |
| p-Crésol à 1 % * | 30 |
| Phénethylol | 130 |
| Polysantol ® [14)] | 50 |
| Essence d'orange Portugal Brésil | 20 |
| Salicylate de benzyle | 100 |
| Salicylate de cis-3-hexénol | 150 |
| Tonalide ® [15)] | 60 |
| Undécalactone gamma à 10% * | 40 |
| Vanilline | 50 |
| Essence d'Ylang extra à 10% * | 50 |
| Total | 3700 |

* dans le dipropylène glycol (DIPG)
4) pentadécanolide ; origine : Firmenich SA, Genève, Suisse
5) 2-méthyl-4-(2,2,3-triméthyl-3'-cyclopentén-1-yl)-4-pentén-1-ol ; origine : Firmenich SA, Genève, Suisse
6) 3-(4-éthyl-1-phényl)-2,2-diméthylpropanal ; origine : International Flavors and Fragrances, USA
7) origine : International Flavors and Fragrances, USA
8) dihydrojasmonate de méthyle ; origine : Firmenich SA, Genève, Suisse
9) 1-(4-méthoxyphényl)-1-éthanone ; origine : Givaudan-Roure SA, Vernier, Suisse
10) méthylionone ; origine : Firmenich SA, Genève, Suisse
11) carbonate de cis-3-héxényle méthyle ; origine : International Flavors and Fragrances, USA
12) 3-(4-ter-buthylphényl)-2-méthylpropanal ; origine : Givaudan-Roure SA, Vernier, Suisse
13) carboxaldéhyde de 4-(4-hydroxy-4-méthylpentyle)-3-cyclohexène ; origine : International Flavors and Fragrances, USA
14) 3,3-diméthyl-5-(2,2,3-triméthyl-3-cyclopentén-1-yl)-4-pentén-2-ol ; origine : Firmenich SA, Genève, Suisse
15) 6-acétyl-1,1,3,4,4,6-hexaméthyl-tétrahydronaphtalène ; oxigine : Polak's Frutal Works

L'addition de 300 parties en poids des composés de formule (I) selon l'invention donne à cet accord de base fleuri-herbacé une très jolie connotation boisée-cuir absolument nouvelle, qui ne peut être rendue par aucune combinaison de produits boisés et de produits à odeur cuir connus de l'art antérieur.

Exemple 3

Composition parfumante

On a préparé une composition parfumante de base pour une eau de toilette masculine de type chyprée-cuir à partir des ingrédients suivants :

| Ingrédients | Parties en poids |
|---|---|
| Acétate de géranyle | 5 |
| Acétate de lynalyle | 70 |
| Essence de bergamote | 560 |
| Essence de citron Sfuma | 60 |
| Coumarine | 50 |
| Dihydromyrcénol | 80 |
| Eugénol | 5 |
| Habanolide ® 1) | 480 |
| Hédione®HC 2) | 100 |
| Indol à 10% * | 25 |
| β-Ionone | 5 |
| Isobutylquinoléïne ® 3) à 10% * | 10 |
| Lyral ®4) | 250 |
| Essence de mandarine Sfuma | 290 |
| Mousse cristal à 10% * | 140 |
| Muscade | 30 |
| trans-1-(2,2,6-triméthyl-1-cyclohexyl)-3-hexanol 5) à 10% * | 55 |
| Huile de patchouli | 70 |
| Salicylate d'amyle | 80 |
| Tonalide ® 6) | 400 |
| Triplal ® 7) à 10% * | 30 |
| Undécalactone gamma à 10% * | 5 |
| Vertofix Coeur 8) | 400 |
| Total | 3200 |

* dans le dipropylène glycol (DIPG)
1) pentadécénolide ; origine : Firmenich SA, Genève, Suisse
2) cis-dihydrojasmonate de méthyle ; origine : Firmenich SA, Genève, Suisse
3) origine : International Flavors and Fragrances, USA
4) 4-(4-hydroxy-4-méthylpentyle)-3-cyclohexèn-1-carboxaldéhyde ; origine : International Flavors and Fragrances, USA
5) origine : Firmenich SA, Genève, Suisse
6) 6-acétyl-1,1,3,4,4,6-hexaméthyl-tétrahydronaphtalène ; origine : Polak's Frutal Works
7) 2,4-diméthyl-3-cyclohexén-1-carboxaldéhyde ; origine : International Flavors and Fragrances, USA
8) origine : International Flavors and Fragrances, USA

L'addition de 1000 parties en poids du composé de formule (I) apporte à cet accord chypré-cuir masculin une jolie connotation cuir très naturelle et renforce l'aspect boisé conféré par le Vertofix. Le parfum devient par ailleurs plus masculin.

Exemple 4

Composition parfumante

On a préparé une composition parfumante de base de type épicé-ylang pour un détergent en poudre à partir des ingrédients suivants :

| Ingrédients | Parties en poids |
|---|---|
| Acétate de verdyle | 40 |
| Alcool cinnamique à 50% * | 70 |
| Aldéhyde anisique | 10 |
| Aldéhyde C 11 undécyclique à 10% * | 20 |

* dans le dipropylèneglycol

(suite)

| Ingrédients | Parties en poids |
|---|---|
| Aldehyde C 12 à 50% * | 40 |
| Aldéhyde cinnamique à 50% * | 10 |
| Aldéhyde phénylacétique à 1% * | 50 |
| Benzoate de méthyle à 10% * | 35 |
| Benzophénone | 100 |
| 4-Cyclohexyl-2-méthyl-2-butanol 1) | 120 |
| Dihydromyrcénol | 135 |
| Habanolide ® 2) à 50% * | 300 |
| Hédione ® 3) | 70 |
| Héliotropine | 20 |
| Indolarome ®4) à 10% * | 50 |
| Iralia® total 5) | 250 |
| Essence de Lavandin Grosso | 50 |
| Méthylisoeugénol | 20 |
| Méthyl-para-crésol | 5 |
| Méthyl octin carbonate à 10% * | 15 |
| Oxyde de rose | 15 |
| Phénéthylol | 200 |
| Phénylhexanol | 100 |
| Polysantol ® 6) | 100 |
| 9-Décén-1-ol | 25 |
| Teipinéol | 70 |
| Vert de Lilas | 5 |
| Ionone alpha | 55 |
| Wardia ® 7) | 20 |
| Total | 2000 |

* dans le dipropylèneglycol
1) origine : Firmenich SA, Genève, Suisse
2) pentadécénolide ; origine : Firmenich SA, Genève, Suisse
3) dihydrojasmonate de méthyle ; origine : Firmenich SA, Genève, Suisse
4) 4,4A,5,9B-tétrahydro-indéno[1,2-D]-1,3-dioxine ; origine : International Flavors & Fragrances, USA
5) méthylionone ; origine : Firmenich SA, Genève, Suisse
6) 3,3-diméthyl-5-(2,2,3-triméthyl-3-cyclopentén-1-yl)-4-pentén-2-ol ; origine : Firmenich SA, Genève, Suisse
7) composition florale ; origine : Firmenich SA, Genève, Suisse

L'adjonction de 400 parties en poids du composé de formule (I) apporte à cet accord la dimension boisée qui lui faisait défaut, augmente nettement son impact sur linge sec et le coté cuir naturel du composé de la présente invention se marie particulièrement bien avec les notes épicées-ylang de cette fragrance.

## Revendications

**1.** Composé de formule

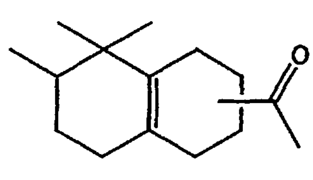

(I)

dans laquelle le groupe substituant acétyle se trouve en position 2 ou 3 du cycle, ou tout mélange de composés de formule (I).

2. Utilisation d'un composé selon la revendication 1 à titre d'ingrédient parfumant.

3. Composition parfumante ou produit parfumé contenant un composé selon la revendication 1 en tant qu'ingrédient parfumant.

4. Produit parfumé selon la revendication 3 sous la forme d'un parfum ou d'une eau de toilette, d'une lotion après rasage, d'une préparation cosmétique, d'un savon, d'un shampoing ou après-shampoing ou autre produit de soin capillaire, d'un gel de bain ou de douche, d'un déodorant corporel ou d'un déodorant ambiant, d'un détergent ou adoucissant textile ou d'un produit d'entretien.

5. Procédé pour la préparation d'un composé selon la revendication 1 **caractérisé en ce qu'**on soumet un composé de formule

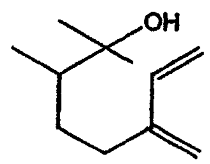

(II)

à une réaction de Diels Aider avec une méthyle vinyle cétone suivie d'une déshydratation et d'une cyclisation.

6. Composé de formule

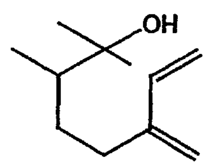

(II)

## Claims

1. A compound of formula

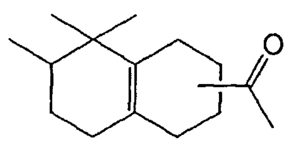

(I)

wherein the acetyl substituting group is either in position 2 or in position 3 of the ring, or any mixtures of compound of formula (I).

2. Use of a compound according to claim 1, as a perfuming ingredient.

3. A perfuming composition or a perfumed product containing as a perfuming ingredient a compound according to claim 1.

4. A perfumed product according to claim 3, in the form of a perfume or a cologne, an after-shave lotion, a cosmetic preparation, a soap, a shampoo or hair-conditioner or other hair-care product, a bath or shower gel, a body or air deodorant, a detergent or a fabric softener, or a household product.

**5.** Process for the preparation of a compound according to claim 1, **characterised in that** a compound of formula

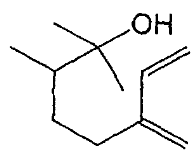

(II)

is subjected to a Diels Alder type reaction in the presence of a methyl vinyl ketone, followed by dehydration and cyclisation reactions.

**6.** A compound of formula

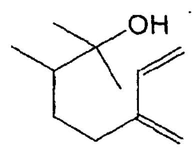

(II)

## Patentansprüche

**1.** Verbindung der Formel

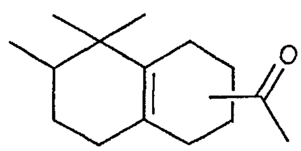

(I)

in der sich die substituierende Acetylgruppe in der Position 2 oder 3 des Rings befindet, bzw. jegliche Mischung von Verbindungen der Formel (I).

**2.** Verwendung einer Verbindung gemäß Anspruch 1 als Riechstoff.

**3.** Duftzusammensetzung oder parfümiertes Produkt, welche(s) als Riechstoff eine Verbindung gemäß Anspruch 1 enthält.

**4.** Parfümiertes Produkt gemäß Anspruch 3 in Form eines Parfüms oder Eau de Toilette, einer After-Shave-Lotion, eines kosmetischen Präparats, einer Seife, eines Shampoo oder eines nach der Haarwäsche anzuwendenden bzw. eines sonstigen Haarpflegeproduktes, eines Bade- oder Duschgels, eines Körper- oder Raumluftdeodorants, eines Detergens oder Textilweichspülers, oder eines Allzweckreinigers.

**5.** Verfahren zur Herstellung einer Verbindung gemäß Anspruch 1, **dadurch gekennzeichnet, daß** eine Verbindung der Formel

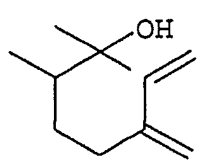

(II)

mit einem Methylvinylketon einer Diels-Alder-Reaktion unterzogen wird, gefolgt von einer Dehydrierung und einer

Cyclisierung.

**6.** Verbindung der Formel

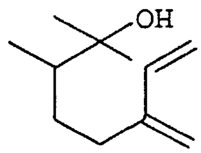

(II)